(19) Europäisches Patentamt
European Patent Office
Office européen des brevets

(11) EP 1 400 504 A1

(12) EUROPÄISCHE PATENTANMELDUNG

(43) Veröffentlichungstag:
**24.03.2004 Patentblatt 2004/13**

(51) Int Cl.[7]: **C07C 45/50**, C07C 47/02

(21) Anmeldenummer: **03020385.5**

(22) Anmeldetag: **10.09.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **19.09.2002 DE 10243446**

(71) Anmelder: **Celanese Chemicals Europe GmbH**
**61476 Kronberg (DE)**

(72) Erfinder:
- **Bohnen, Hans**
  **47441 Moers (DE)**
- **Herwig, Jürgen**
  **D-46569 Hünxe (DE)**
- **Hoff, Dietmar**
  **D-67122 Altrip (DE)**
- **Van Hal, Roy**
  **NL-6451 HD Schinveld (NL)**
- **Wasserscheid, Peter**
  **D-91054 Erlangen (DE)**

(54) **Verfahren zur Herstellung von Aldehyden**

(57) Die vorliegende Erfindung betrifft ein Hydroformylierungsverfahren durch Umsetzung von Olefinen oder olefinisch ungesättigten Verbindungen in Gegenwart mindestens einer Rhodiumverbindung und in Gegenwart von sulfonierten Arylphosphinen in ionischen Flüssigkeiten, die aus einem quatären einfach geladenen Ammoniumion oder aus dem Äquivalent eines mehrfach geladenen Ammoniumions und aus organischen Sulfonaten oder Sulfaten aufgebaut sind.

EP 1 400 504 A1

## Beschreibung

Die vorliegende Erfindung betrifft ein Verfahren zur Herstellung von Aldehyden durch Umsetzung von Olefinen oder olefinisch ungesättigten Verbindungen mit Wasserstoff und Kohlenmonoxid (Hydroformylierung) in Gegenwart von Rhodium oder Rhodiumverbindungen, sulfonierten Arylphosphinen und einer nicht wäßrigen ionischen Flüssigkeit der allgemeinen Formel $(Q^{\oplus})_a A^{a-}$. In dieser Formel steht $Q^{\oplus}$ für ein einfach geladenes, gegebenenfalls durch organische Reste substituiertes Ammonium-Kation oder das Äquivalent eines mehrfach geladenen, gegebenenfalls durch organische Reste substituierten Ammonium-Kations und $A^{a-}$ für ein organisches Sulfonat oder organisches Sulfat. a ist eine ganze Zahl, mindestens gleich 1, und beschreibt die Ladung des Anions bzw. die Anzahl der Kationen mit der Ladung +1 in den der allgemeinen Formel entsprechenden Verbindungen.

Aldehyde besitzen als wertvolle Zwischenprodukte in der technischen Chemie eine große wirtschaftliche Bedeutung. Aus ihnen lassen sich z.B. Alkohole, Carbonsäuren und Amine herstellen, die wiederum als Ausgangsstoffe für die Herstellung wichtiger Endprodukte verwendet werden.

Die Hydroformylierung gehört zu den industriell in großem Umfang ausgeübten Verfahren. Die Reaktion wird durch Hydridometallcarbonyle, vorzugsweise solcher der Metalle der Gruppe VIII des Periodensystems der Elemente, katalysiert. Während zunächst ausschließlich Kobalt als Katalysatormetall technische Anwendung fand, gewinnen nunmehr Prozesse, die Rhodium als Katalysatormetall einsetzen, zunehmende Bedeutung.

Die Herstellung von Aldehyden durch Hydroformylierung von Olefinen kann einphasig in einer organischen Phase erfolgen. Dabei liegt der Katalysator, z.B. ein Rhodium/Triphenylphosphinkomplex, gelöst in dem aus Ausgangsstoffen und Reaktionsprodukt gebildeten Reaktionsgemisch vor. Zusätzlich kann ein organisches Lösungsmittel, z.B. Toluol, Xylol oder Tetrahydrofuran, zugegen sein.

Probleme wirft bei diesem Verfahren die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und der daraus resultierenden Bildung von Nebenprodukten zu Lasten der Aldehydausbeute aber nur bei der Hydroformylierung niedriger Olefine, d.h. Olefinen mit bis zu etwa 5 Kohlenstoffatomen im Molekül, beschritten werden. Außerdem kann die thermische Belastung des Destillationsgutes zu erheblichen Verlusten an Katalysator durch Zersetzung der katalytisch wirksamen Komplexverbindungen führen.

Diese Mängel lassen sich vermeiden, wenn die Hydroformylierungsreaktion in einem Zweiphasensystem durchgeführt wird. Ein derartiges Verfahren ist z.B. in der DE-PS 26 27 354 beschrieben. Dieser Prozeß ist charakterisiert durch das Vorliegen einer organischen Phase, die die Ausgangsolefine und das Reaktionsprodukt enthält und einer wäßrigen Phase, in der der Katalysator gelöst ist. Als Katalysatoren werden wasserlösliche Rhodiumkomplexverbindungen eingesetzt, die wasserlösliche Phosphine als Liganden enthalten. Zu den Phosphinen zählen insbesondere Triarylphosphine, Trialkylphosphine und arylierte bzw. alkylierte Diphosphine, deren organische Reste durch Sulfonsäuregruppen oder Carboxylgruppen substituiert sind. Ihre Herstellung ist z.B. aus DE-PS 26 27 354 bekannt.

Der in Gegenwart einer wäßrigen katalysatorhaltigen Phase zweiphasig durchgeführte Hydroformylierungsprozeß bewährt sich besonders bei der Hydroformylierung niedriger Olefine, insbesondere bei Ethylen und Propylen. Setzt man hingegen höhere Olefine wie Hexen, Octen oder Decen ein, so geht der Umsatz deutlich zurück. Die Minderung des Umsatzes ist wohl auf die Abnahme der Löslichkeit höherer Olefine in Wasser zurückzuführen, da man annimmt, daß die Reaktion zwischen den Reaktanten in der wäßrigen Phase abläuft. Für diese Hypothese spricht, daß der Olefinumsatz deutlich erhöht wird, wenn man der wäßrigen Katalysatorlösung ein Phasentransferreagenz (Lösungsvermittler) zusetzt. Bewährt haben sich nach EP-B-0 562 451 insbesondere kationische Lösungsvermittler der allgemeinen Formel $[A\text{-}N(R^1R^2R^3)]^+ E^-$, in der A für einen geradkettigen oder verzweigten Alkylrest mit 6 bis 25 Kohlenstoffatomen steht, $R^1$, $R^2$, $R^3$ gleich oder verschieden sind und geradkettige oder verzweigte Alkylreste mit 1 bis 4 Kohlenstoffatomen bedeuten und $E^-$ für ein Anion, insbesondere Sulfat, Tetrafluoroborat, Acetat, Methosulfat, Benzolsulfonat, Alkylbenzolsulfonat, Toluolsulfonat, Lactat oder Citrat steht.

Neben der ausreichenden Löslichkeit des Olefins in der wäßrigen Phase ist für die zweiphasige Durchführung des Hydroformylierungsprozesses in Gegenwart einer wäßrigen katalysatorhaltigen Phase eine ausreichenden Stabilität des umzusetzenden Olefins gegenüber Wasser notwendig. Wasserempfindliche Olefine, wie z.B. Acrylsäureester oder ungesättigte Acetale, lassen sich daher nach diesem Verfahren nicht erfolgreich einsetzen.

Seit jüngster Zeit rücken sogenannte ionische Flüssigkeiten als Lösungsmittel für den Katalysator in den Mittelpunkt. Bei den ionischen Flüssigkeiten handelt es sich um niedrig schmelzende Salze, die unterhalb einer Temperatur von 100°C flüssig sind und die praktisch keinen Dampfdruck aufweisen. Ionische Flüssigkeiten können als Lösungsmittel für einen katalytisch aktiven Übergangsmetallkomplex und im Überschuß vorhandener Liganden dienen und sie bilden häufig eine von der organischen Produktphase separierte Phase. Die Verwendung von ionischen Flüssigkeiten gestattet daher ebenfalls eine zweiphasige Reaktionsführung unter Vermeidung von Wasser und nach erfolgter Reaktion können die organische Produktphase und die ionische Flüssigkeit, die den katalytischen Übergangsmetallkomplex gelöst enthält, in einfacher Weise mittels Phasentrennung voneinander getrennt werden.

Mittels ionischer Flüssigkeiten lassen sich Zweiphasenprozesse unter nicht wäßrigen Bedingungen durchführen. Eigenschaften, Herstellung und Anwendung ionischer Flüssigkeiten werden in den Übersichtsartikeln in Angew. Chem. 2000, 112, 3926-3945 und Chem. Commun., 2001, 2399-2409, ausführlich erläutert.

CHEMTECH, September 1995, Seiten 26 bis 30 beschreibt katalytische Reaktionen in einem nicht wäßrigen Zweiphasensystem. Hiernach werden nicht wäßrige ionische Flüssigkeiten, die schon bei Raumtemperatur flüssig sind, z.B. eine Mischung aus 1,3-Dialkylimidazolium Chlorid, bevorzugt 1-n-Butyl-3-methylimidazolium Chlorid (abgekürzt $[BMI]^+[Cl]^-$), und Aluminiumchlorid und/oder Ethylaluminiumdichlorid, als Lösungsmittel für den Katalysator verwendet. Als Beispiele für mit derartigen Katalysatorlösungen durchgeführte Reaktionen sind die Olefindimerisierung in Gegenwart von Nickelkomplexverbindungen, z.B. die Propendimerisierung zu isomeren Hexenen oder die Butendimerisierung zu Iso-Octenen zu nennen. Bei diesen Umsetzungen fällt das Reaktionsprodukt als obere Phase an, während die katalysatorhaltige nicht wäßrige ionische Flüssigkeit die untere Phase bildet und durch einfache Phasentrennung isoliert werden kann. Die Lösung des Katalysators in der nicht wäßrigen ionischen Flüssigkeit kann erneut in den Prozeß eingeführt werden.

Aus Am. Chem. Soc., Div. Pet. Chem. 1992, 37, Seiten 780 bis 785 ist bekannt, daß eine nicht wäßrige ionische Flüssigkeit, bestehend aus $[BMI]^+[Cl]^-$ und Aluminiumchlorid als Lösungsmittel dient, in dem nach Zusatz von Ethylaluminiumdichlorid und $NiCl_2(PR_3)_2$, mit R gleich Isopropyl, die Dimerisierung von Propen erfolgt.

Die Verwendung von niedrig schmelzenden Phosphoniumsalzen, z.B. von Tetrabutylphosphonium-Bromid als Lösungsmittel in Hydroformylierungsreaktionen wird im Journal of Molecular Catalysis, 47 (1988) Seiten 99 - 116 beschrieben. Danach führt die Hydroformylierung von Olefinen, z.B.Octen-1, mit Rutheniumcarbonylkomplexen in Gegenwart von stickstoff- oder phosphorhaltigen Liganden, z.B. von 2,2'-Dipyridin oder 1,2-Bis(diphenylphosphino)ethan, bei Temperaturen von 120 bis 180 °C zu einem Gemisch aus n-Nonanol und n-Nonanal. Bei diesem Verfahren erhält man ein Reaktionsgemisch mit einem n-Nonanol-Anteil von bis zu 69 Gew.-%, bezogen auf das Reaktionsgemisch. Die Isolierung des gewünschten n-Nonanals erfordert daher einen erheblichen Destillationsaufwand.

Die europäische Patentanmeldung EP-A-0 776 880 offenbart die Hydroformylierung von Olefinen in Gegenwart von quaternären Ammonium- und/oder Phosphoniumsalzen als Lösungsmittel für den Katalysator. Bevorzugt werden Salze, die $[BMI]^+$ als Kation enthalten.

Auch Salze von quaternären Diaminen, bei denen das Kation die allgemeine Formel

$$R^1R^2N^{\oplus}=CR^3\text{-}R^5\text{-}R^3C=N^{\oplus}R^1R^2$$

hat, wobei $R^1$, $R^2$, $R^3$ gleich oder verschieden sind und Wasserstoff oder einen Kohlenwasserstoffrest mit 1 bis 1 2 Kohlenstoffatomen bedeuten u nd $R^5$ ein Alkylenrest, z.B. Methylen ($-CH_2-$) , Ethylen ($-CH_2-CH_2-$), Propylen ($-CH_2-CH_2-CH_2-$) oder ein Phenylenrest, ist, werden als Lösungsmittel für Hydroformylierungskatalysatoren verwendet. Geeignete Anionen sind beispielsweise Hexafluorophosphat, Hexafluoroantimonat, Tetrachloroaluminat oder Tetrafluoroborat. Diese quaternären Ammonium- und/oder Phosphoniumsalze sind bereits unterhalb von 90°C, vorzugsweise unterhalb von 85°C und besonders bevorzugt unterhalb von 50°C flüssig.

Der in den genannten Lösungsmitteln gelöste Hydroformylierungskatalysator enthält als aktives Metall Kobalt, Rhodium, Iridium, Ruthenium, Palladium oder Platin und als Ligand ein tertiäres Phosphin oder tertiäres sulfoniertes Phosphin, ein tertiäres Arsin, tertiäres Stibin oder ein Phosphit. Das Molverhältnis von Ligand zu Metall beträgt 9,5.

Die katalytisch wirksamen Metalle werden als Verbindungen, Rhodium z.B. in Form von Rhodiumacetylacetonatdicarbonyl oder Rhodiumcarbonyl $Rh_6(CO)_{16}$, eingesetzt. Aus ihnen bildet sich unter den Reaktionsbedingungen der Hydroformylierungskatalysator. Die Hydroformylierungsreaktion wird besonders bevorzugt zwischen 30 und 90°C durchgeführt.

Auch nach Angew. Chem. 1995, 107 Nr.23/24 Seiten 2941 bis 2943 lassen sich Hydroformylierungsreaktionen unter Verwendung bei Raumtemperatur flüssiger 1,3 Dialkylimidazoliumsalze als katalysatorhaltiges, nicht mit dem organischen Reaktionsgemisch mischbares Lösungsmittel durchführen. Hierzu wird Rhodiumdicarbonylacetylacetonat als Katalysatorvorstufe zu einer Lösung von Triphenylphosphin in $[BMI]^{\oplus}$ $[PF_6]^{\ominus}$ gegeben, das Molverhältnis Phosphor (III) zu Rhodium kann von 3 bis 10 variieren. Der Katalysator wird mit Synthesegas (Volumenverhältnis Wasserstoff zu Kohlenmonoxid gleich 1:1) präformiert. Anschließend setzt man n-Penten-1 mit Synthesegas gleicher Zusammensetzung bei einer Temperatur von 80°C um. Auch in diesem Falle läßt sich die organische Produktphase in einfacher Weise von der katalysatorhaltigen, nicht wäßrigen ionischen Flüssigkeit durch Dekantieren abtrennen.

Nachteilig bei den bekannten Verfahren ist neben dem Austrag des P hosphinliganden der Austrag des katalytisch aktiven Metalls aus der nicht wäßrigen ionischen Flüssigkeit in die organische Phase. Nach dem Stand der Technik läßt sich dieser Nachteil umgehen, wenn an Stelle von neutralen Liganden, wie Triphenylphosphin, geladene Liganden, z.B. mono- oder trisulfoniertes Triphenylphosphin, verwendet werden, da zu erwarten ist, daß geladene Liganden die Löslichkeit der katalytisch aktiven Metallverbindungen in der nicht wäßrigen ionischen Flüssigkeit erhöhen. Zwar konnte durch Verwendung geladener Liganden der Austrag des katalytisch aktiven Metalls reduziert werden,

gleichzeitig verringerte sich jedoch die Aldehydausbeute auf nur noch 16 bis 33 % (Angew. Chem. 1995, 107 Nr.23/24 Seiten 2941 bis 2943, EP-A-0 776 880).

Bei der aus Chem. Commun. 2001, 451-452 bekannten Variante erhöht man die Polarität der Liganden durch das Einführen der positiv geladenen Guanidiniumgruppe an den aromatischen Rest.

Die bisher bekannten ionischen Flüssigkeiten beruhen in überwiegendem Maße auf niedrig schmelzenden Salzen mit Ammonium- oder Phosphoniumkationen und komplexen Anionen, die Halogene, wie Fluor oder Chlor enthalten.

Auf Grund des korrosiven Verhaltens halogenhaltiger Schmelzen gegenüber aus metallischen Werkstoffen gefertigten Reaktionsgefäßen besteht bei dem technischen Einsatz solcher Salzschmelzen die Gefahr einer verstärkten korrosiven Beanspruchung der metallischen Reaktorgefäße.

Zudem ist die Entsorgung der nach Erschöpfung des Katalysatorsystems anfallenden halogenhaltigen Salzschmelze besonders aufwendig.

Es bestand daher die Aufgabe ein Verfahren zur Hydroformylierung von Olefinen oder olefinisch ungesättigten Verbindungen in Gegenwart eines katalytisch wirkenden Metalls und einer nicht wäßrigen ionischen Flüssigkeit zu entwickeln, das die beschriebenen Nachteile vermeidet.

Die Erfindung besteht in einem Verfahren zur Herstellung von Aldehyden durch Umsetzung von Monoolefinen, konjugierten und nichtkonjugierten Polyolefinen, Cycloolefinen oder Derivaten dieser Verbindungsklassen mit Kohlenmonoxid und Wasserstoff bei Temperaturen von 20 bis 150°C und Drücken von 0,1 bis 20 MPa in Gegenwart einer nicht wäßrigen ionischen Flüssigkeit der allgemeinen Formel $(Q^{\oplus})_a A^{a-}$ und mindestens einer Rhodiumverbindung und mindestens eines sulfonierten Arylphosphins. Es ist dadurch gekennzeichnet, daß $Q^{\oplus}$ ein einfach geladenes, gegebenenfalls durch organische Reste substituiertes Ammonium-Kation oder das Äquivalent eines mehrfach geladenen, gegebenenfalls durch organische Reste substituierten Ammonium-Kations ist und $A^{a-}$ für ein organisches Sulfonat oder organisches Sulfat der allgemeinen Formeln

$$R^1 \leftarrow\!(SO_3^-)_a \text{ oder } R^2 \leftarrow\!(OSO_3^-)_a \qquad (1)$$

steht, wobei $R^1$ und $R^2$ einen geradkettigen oder verzweigten Alkylrest mit 1-20 Kohlenstoffatomen, einen Alkylarylrest mit 7-20 Kohlenstoffatomen, einen Arylrest mit 6-14 Kohlenstoffatomen, einen Cycloalkylrest mit 3-7 Kohlenstoffatomen oder einen Aralkylrest mit 7-20 Kohlenstoffatomen bedeutet; oder $R^1$ einen Rest der allgemeinen Formel

$$R^3\text{-O}\{(CH_2\}_n O\}_m \qquad (1a)$$

bedeutet, in der $R^3$ einen geradkettigen oder verzweigten Alkylrest mit 1-20 Kohlenstoffatomen, einen Alkylarylrest mit 7-20 Kohlenstoffatomen, einen Arylrest mit 6-14 Kohlenstoffatomen, einen Cycloalkylrest mit 3-7 Kohlenstoffatomen oder einen Aralkylrest mit 7-20 Kohlenstoffatomen darstellt, n eine ganze Zahl von 2-12 bedeutet und m ganzzahlige Werte von 1-100 annimmt; und a eine ganze Zahl ist und die Anzahl der an dem organischen Rest gebundenen Sulfon- oder Sulfatreste bedeutet und mindestens gleich 1 ist.

Überraschenderweise wurde gefunden, daß sich in den erfindungsgemäß eingesetzten nicht wäßrigen ionischen Flüssigkeiten gelöste Rhodiumverbindungen hervorragend für die Hydroformylierung von Olefinen oder olefinisch ungesättigten Verbindungen eignen.

Besonders bewährt in dem erfindungsgemäßen Hydroformylierungsverfahren haben sich solche ionischen Flüssigkeiten der allgemeinen Formel (1), in denen $R^1$ oder $R^2$ für einen geradkettigen oder verzweigten Alkylrest mit 1-12 Kohlenstoffatomen, einen Alkylarylrest mit 7-12 Kohlenstoffatomen, einen Arylrest mit 6-10 Kohlenstoffatomen, einen Cycloalkylrest mit 5-7 Kohlenstoffatomen oder einen Aralkylrest mit 7-12 Kohlenstoffatomen steht, $R^3$ einen geradkettigen oder verzweigten Alkylrest mit 1-12 Kohlenstoffatomen, einen Alkylarylrest mit 7-12 Kohlenstoffatomen, einen Arylrest mit 6-10 Kohlenstoffatomen, einen Cycloalkylrest mit 5-7 Kohlenstoffatomen oder einen Aralkylrest mit 7-12 Kohlenstoffatomen bedeutet, n gleich 2, 3 oder 4 ist, m eine ganze Zahl zwischen 1-50 ist; und a gleich 1 oder 2 ist.

Insbesondere hat sich die Verwendung der Anionen Octylsulfat und Tosylat als geeignet erwiesen.

Ionische Flüssigkeiten auf Basis der sulfon- oder sulfathaltigen Anionen zeichnen sich im Vergleich zu den bekannten ionischen Flüssigkeiten, die komplexe Anionen mit Halogenen als Bestandteile enthalten, wie zum Beispiel das Hexafluorophosphat- oder Tetrafluoroborat-Anion, durch ihre abgeschwächte korrosive Wirkung gegenüber metallischen Apparateteilen aus. Als Folge der geringeren korrosiven Beanspruchung sinkt die Reparaturanfälligkeit der verwendeten Apparateteile, was sich vorteilhaft auf die Wirtschaftlichkeit des Hydroformylierungsprozesses auswirkt. Zudem können bei Verwendung der ionischen Flüssigkeiten der allgemeinen Formel (1) Hydroformylierungsreaktionen in solchen Reaktionsgefäßen durchgeführt werden, die aus weniger korrosionsresistenten Werkstoffen gefertigt sein

müssen. Die Verwendung solcher preisgünstiger Werkstoffe für die Reaktionsgefäße hat einen besonders hohen wirtschaftlichen Wert, weil sich dadurch die Investitionskosten senken lassen.

Zudem wurde überraschenderweise gefunden, dass sich bei Verwendung der Anionen der allgemeinen Formel (1) in der ionischen Flüssigkeit bei Hydroformylierungsprozessen höhere Umsatz- und Selektivitätswerte zu den geradkettigen Aldehyden erzielen lassen im Vergleich zu Hydroformylierungsprozessen, die in herkömmlichen ionischen Flüssigkeiten unter Verwendung halogenhaltiger komplexer Anionen durchgeführt werden. Ohne eine theoretische Deutung für diesen überraschenden Effekt geben zu wollen, kann vermutet werden, dass die in den Anionen der allgemeinen Formel (1) gebundenen organischen Reste die Löslichkeit der olefinisch ungesättigten Verbindungen in der ionischen Flüssigkeit erhöhen.

Als Kationen $Q^{\oplus}$ enthalten die in dem erfindungsgemäßen Hydroformylierungsprozeß verwendeten ionischen Flüssigkeiten insbesondere durch organische Reste substitutierte Ammoniumionen, nämlich Ammoniumionen, die s ich von M ono- oder Diaminen ableiten. D ie Ammoniumionen von Monoaminen entsprechen den allgemeinen Formeln (2) und (3)

$$^{\oplus}NR^4R^5R^6R^7 \qquad (2)$$

und

$$R^4R^5N^{\oplus}=CR^6R^7 \qquad (3)$$

wobei $R^4$, $R^5$, $R^6$ und $R^7$ gleich oder verschieden sind und Wasserstoff, insbesondere mit der Maßgabe, daß mindestens ein $R^4$, $R^5$, $R^6$, $R^7$ nicht Wasserstoff ist, oder einen linearen oder verzweigten, aliphatischen Kohlenwasserstoffrest mit 1 bis 20 Kohlenstoffatomen, einen cycloaliphatischen oder aromatischen Kohlenwasserstoffrest mit 6 bis 20 Kohlenstoffatomen oder einen Alkoxyrest mit 1 bis 10 Kohlenstoffatomen bedeuten. Beispiele für solche Reste sind Alkyl-, Alkenyl-, Cycloalkyl-, Aryl- Alkylaryl-, oder Aralkylreste.

Als Kationen der ionischen Flüssigkeiten kommen ferner Ionen in Betracht, die sich von gesättigten oder ungesättigten cyclischen Verbindungen sowie von aromatischen Verbindungen mit jeweils einem dreibindigen N-Atom im 4-bis 10-, vorzugsweise 5- bis 6-gliedrigen heterocyclischen Ring ableiten. Solche Kationen lassen sich vereinfacht (d.h. ohne Angabe von genauer Lage und Anzahl der Doppelbindungen im Molekül) durch die nachstehenden allgemeinen Formeln (4) und (5) wiedergeben.

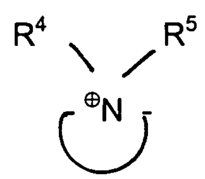

(4)

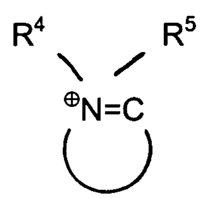

(5)

$R^4$ und $R^5$ besitzen dabei die vorgenannte Bedeutung. Beispiele für cyclische Amine der vorgenannten Art sind Pyrrolidin, Dihydropyrrol, Pyrrol, Indol, Carbazol, Piperidin, Pyridin, die isomeren Picoline und Lutidine, Chinolin und i-Chinolin.

Bevorzugte Kationen gehen auf aliphatische, cycloaliphatische oder aromatische Diamine zurück. Sie folgen den allgemeinen Formeln (6) und (7)

$$R^4R^5R^6N^{\oplus}\text{-G-}N^{\oplus}R^7R^8R^9 \qquad (6)$$

$$R^4R^5N^{\oplus} = CR^6\text{-G-}R^6C = N^{\oplus}R^4R^5 \qquad (7)$$

in denen $R^4$, $R^5$, $R^6$, $R^7$, $R^8$ und $R^9$ gleich oder verschieden sind und Wasserstoff, einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 30 Kohlenstoffatomen, einen Alkylaryl- oder Aralkylrest mit 7 bis 40 Kohlenstoffatomen oder einen Alkoxyrest mit 1 bis 10 Kohlenstoffatomen bedeuten. G steht für einen Alkylenrest $(\text{-CHR}^{10}\text{-})_d$, wobei $R^{10}$ Wasserstoff oder ein Kohlenwasserstoffrest mit 1 bis 5 Kohlenstoffatomen und d eine ganze Zahl von 1 bis 8, vorzugsweise 2 bis 6 ist, für einen Arylenrest mit 6 bis 30 Kohlenstoffatomen oder für einen Alkylenarylrest mit 7 bis 40 Kohlenstoffatomen. Beispiele für die durch $R^4$, $R^5$, $R^6$, $R^7$, $R^8$ und $R^9$ bezeichneten Kohlenwasserstoffreste sind Alkyl-, Alkenyl-, Cycloalkyl-, Aryl, Alkylaryl-, oder Aralkylreste, wie Methyl, Ethyl, Propyl, i-Propyl, Butyl, sek.-Butyl, t-Butyl, Amyl, Methylen, Ethyliden, Phenyl, Benzyl. $R^{10}$ wird beispielhaft durch den Methyl-, Ethyl-, n-Propyl-, i-Propylrest und die isomeren Butylreste beschrieben. Beispiele für G sind die Reste Methylen, Ethylen, Propylen, Butylen, 1,4-Phenylen, 1,4-Tolylen, 1,4-Xylylen, 1,1'-Biphenyl-4,4'-diyl, 1,4-Naphthylen, 1,1-Binaphthyl-2,2'-diyl.

Weitere bevorzugte Kationen der erfindungsgemäßen nicht wäßrigen ionischen Flüssigkeiten leiten sich von 1-Amino-3-dialkylaminopropanen der allgemeinen Formel (8)

$$R^{11}R^{12}N\text{-}CH_2\text{-}CH_2\text{-}CH_2\text{-}NH_2 \qquad (8)$$

als Diaminen ab, in der $R^{11}$ und $R^{12}$ gleiche oder verschiedene lineare oder verzweigte Alkylreste mit 4 bis 20 Kohlenstoffatomen sind und beispielsweise n-Butyl, n-Pentyl, n-Hexyl, n-Heptyl, i-Heptyl, n-Octyl, i-Octyl, n-Nonyl, i-Nonyl, n-Decyl, i-Decyl, n-Undecyl, i-Undecyl, n-Dodecyl oder i-Dodecyl bedeuten.

Weitere vorteilhafte Kationen der erfindungsgemäßen nicht wäßrigen ionischen Flüssigkeiten gehen auf folgende Amine zurück: 1-Amino-3-(di-nheptyl)-aminopropan, 1-Amino-3-(di-i-heptyl)-aminopropan, 1-Amino-3-(di-noctyl)-aminopropan, 1-Amino-3-(di-i-octyl)-aminopropan, 1-Amino-3-(di-nnonyl)-aminopropan, 1-Amino-3-(di-i-nonyl)-aminopropan, 1-Amino-3-(di-nundecyl)-aminopropan, 1-Amino-3-(di-i-undedyl)-aminopropan, 1-Amino-3-(di-n-dodecyl)-aminopropan oder 1-Amino-3-(di-i-dodecyl)-aminopropan.

Die vorstehend beschriebenen 1-Amino-3-dialkylaminopropane sind leicht aus N,N-(dialkyl)aminen und Acrylnitril zugänglich (vgl. Ullmanns Encyclopedia of Industrial Chemistry, Vol. A2, 1985).

Schließlich zählen zu den Diaminen, die besonders geeignete Kationen für die ionischen Flüssigkeiten ergeben, auch heterocyclische Verbindungen. Zu ihnen zählen gesättigte oder ungesättigte sowie aromatische Verbindungen mit jeweils zwei dreibindigen N-Atomen im 4- bis 10-, vorzugsweise 5- oder 6-gliedrigen heterocyclischen Ring. Diese Verbindungen können sowohl an den Kohlenstoffatomen als auch an den Stickstoffatomen substituiert sein, vorzugsweise durch Alkylreste mit 1 bis 10 Kohlenstoffatomen und durch Phenylreste. Sie können weiterhin durch, gegebenenfalls substituierte, Benzolringe und/oder Cyclohexanringe unter Ausbildung mehrkerniger Strukturen anelliert sein. Beispiele für solche Verbindungen sind Pyrazol, 3,5-Dimethylpyrazol, Imidazol, Benzimidazol, Dihydropyrazol, Pyrazolidin, Pyridazin, Pyrimidin, Pyrazin, 2,3-, 2,5- und 2,6-Dimethylpyrazin, Cimolin, Phthalazin, Chinazolin, Phenazin und Piperazin. Insbesondere vom Imidazol und seinen Alkyl- und Phenylderivaten abgeleitete Kationen der allgemeinen Formel (9)

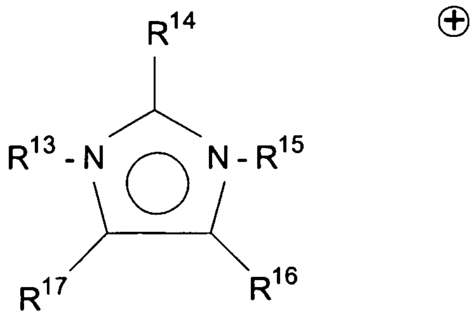

(9)

haben sich als Bestandteil der neuen ionischen Flüssigkeiten bewährt. In dieser Formel sind $R^{13}$, $R^{14}$, $R^{15}$, $R^{16}$ und $R^{17}$ gleich oder verschieden. Sie stehen für Wasserstoff, einen $C_1$- bis $C_{30}$-Alkylrest, einen $C_6$- bis $C_{40}$-Arylrest, einen $C_7$- bis $C_{40}$-Alkylarylrest oder einen $SiR_3{}^{18}$-rest, in dem $R^{18}$ einen $C_1$bis $C_{30}$-Alkylrest oder einen $C_6$- bis $C_{40}$-Arylrest bedeutet. Beispiele für solche Kationen sind: 1-Ethyl-3-methyl-2,4,5-H-imidazolium, 1-Propyl-3-methyl-2,4,5-H-imidazolium, 1-Butyl-3-methyl-2,4,5-H-imidazolium, 1-Butyl-3-ethyl-2,4,5-H-imidazolium, 1,3,4,5-Tetramethyl-2-H-imidazolium, 2,4,5-Trimethyl-1,3-H-imidazolium, 1,2,3,4,5-Pentamethylimidazolium, 1,2,3,5-Tetramethyl-4-H-imidazolium, 1, 2,3,4-Tetramethyl-5-H-imidazolium, 1,3,4,5-Tetraphenyl-2-H-imidazolium, 1,3-Dimethyl-4,5-diphenyl-2-H-imidazolium, 1-Ethyl-3-isopropyl-2,4,5-H-imidazolium, 1-Butyl-3-octanyl-2,4,5-H-imidazolium, 1-Propyl-3-octanyl-2,4,5-H-imidazolium, 1-Ethyl-3-octanyl-2,4,5-H-imidazolium, 1-Methyl-3-octanyl-2,4,5-H-imidazolium, 1,3-Diisoproypl-4,5-dimethyl-2-Himidazolium, 1,4,5-Trimethyl-3-trimethylsilyl-2-H-imidazolium, 2-Ethyl-4-methyl-1,3,5-H-imidazolium, 1,3-Adamantyl-4,5-dimethyl-1-H-imidazolium, 1,2,4,5-Tetramethyl-3-H-imidazolium, 1-Methyl-2,3,4,5-H-imidazolium, 1,3-Dimethyl-2,4,5-H-imidazolium, 2-Methyl-4,5-ethyl-1,3-H-imidazolium, 2,4,5-Trimethyl-1,3-H-imidazolium, 1-Ethyl-2,3, 4,5-H-imidazolium, 1,3-Diethyl-4,5-dimethyl-2-H-imidazolium, 1,3-Diphenyl-4,5-dimethyl-2-H-imidazolium, 1,3-Diphenyl-2,4,5-H-imidazolium, 1,3-Dimethoxy-4,5-dimethyl-2-H-imidazolium, 1-Trimethylsilyl-2,3,5-trimethyl-4-H-imidazolium.

Weiterhin haben sich ionische Flüssigkeiten auf Basis der sulfon- oder sulfathaltigen Anionen der allgemeinen Formel (1) sehr bewährt, deren Kationen sich von Polyaminen ableiten. Beispiele für solche Polyamine sind Hexamethylentetramin und Purin sowie dessen Derivate.

Die ionische Flüssigkeit ist in der organischen Produktphase, die nicht reagiertes Olefin, Aldehyde und Nebenprodukte, beispielsweise Alkohole enthält, nicht löslich. Sie dient als Lösungsmittel für den katalytisch wirksamen Rhodiumkomplex und bildet eine von dem organischen Produkt separierte flüssige Phase. Als Liganden für den Rhodiumkomplex verwendet man sulfonierte Arylphosphine als zur Komplexbildung befähigte Verbindungen, die den Rhodiumkomplex in der ionischen Flüssigkeit gut lösen und einen Rhodiumaustrag in die organische Phase möglichst unterbinden.

Als sulfonierte Arylphosphine eignen sich in dem erfindungsgemäßen Verfahren sulfonierte Triarylphosphine der allgemeinen Formel (10)

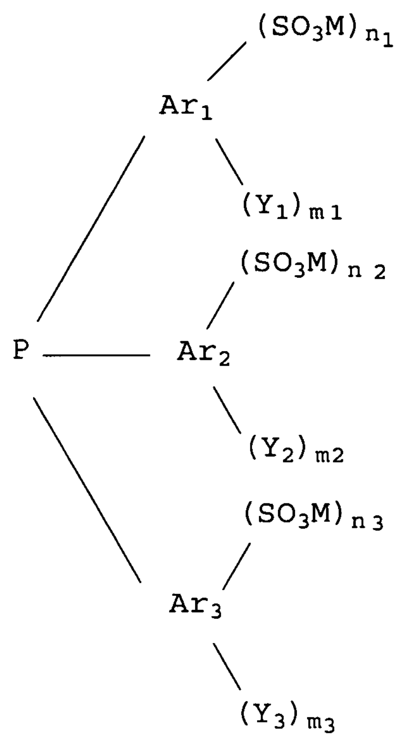

(10)

in der $Ar^1$, $Ar^2$ und $Ar^3$ gleiche oder verschiedene Arylgruppen mit 6 bis 14 Kohlenstoffatomen bedeuten, die Substituenten $Y_1$, $Y_2$ und $Y_3$ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel $NR^{18}R^{19}$ stehen, in der die Substituenten $R^{18}$ und $R^{19}$ gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der $m_1$, $m_2$ und $m_3$ gleich oder verschieden sind und ganze Zahlen von 0 bis 5 bedeuten, in der $n_1$, $n_2$ und $n_3$ gleich oder verschieden sind und für ganze Zahlen von 0 bis 3 stehen, wobei mindestens eine der Zahlen $n_1$, $n_2$ und $n_3$ gleich oder größer 1 ist.

Zu den Triarylphosphinen zählen bevorzugt solche Triarylphosphine, bei denen die Gruppen $Ar^1$, $Ar^2$, $Ar^3$ Phenylgruppen sind; $Y_1$, $Y_2$ und $Y_3$ für die Methyl-, die Ethylgruppe, für die Methoxy-, Ethoxygruppe und/oder für ein Chloratom stehen; und die kationischen Reste M anorganische Kationen von Natrium, Kalium, Calcium und Barium sind. Insbesondere geeignet sind solche Triarylphosphine, bei denen $Ar^1$, $Ar^2$, $Ar^3$ jeweils eine Phenylgruppe bedeuten, $m_1$, $m_2$, $m_3$ gleich 0 sind, $n_1$, $n_2$ und $n_3$ gleich 0 oder 1 sind und $n_1+n_2+n_3$ zusammen 1 bis 3 ausmachen, und bei denen die Sulfonat-Gruppen in meta-Stellung stehen.

Ein für die Durchführung des erfindungsgemäßen Hydroformylierungsverfahrens geeignetes Gemisch an (Sulfophenyl)-diphenylphosphin, Di-(sulfophenyl)phenylphosphin und Tri(sulfophenylphosphin) fällt bei der Sulfonierung von Triphenylphosphin an, wie z.B. aus DE-OS 26 27 354 bekannt. Im Stand der Technik wird (Sulfophenyl) diphenylphosphin als TPPMS, Di-(sulfophenyl)phenylphosphin als TPPDS und Tri(sulfophenyl)phosphin als TPPTS abgekürzt.

Als sulfonierte Arylphosphine eignen sich ebenfalls sulfonierte Diphosphine der allgemeinen Formeln (11) oder (12)

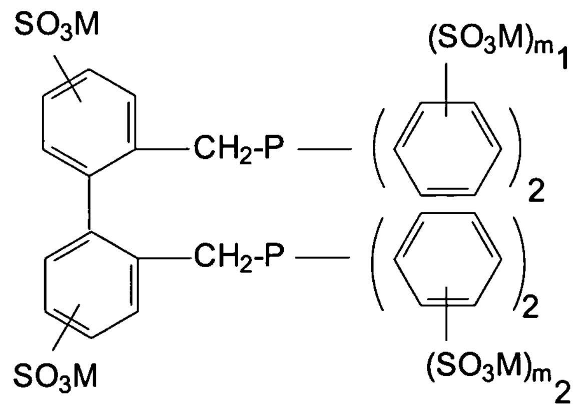

(11)

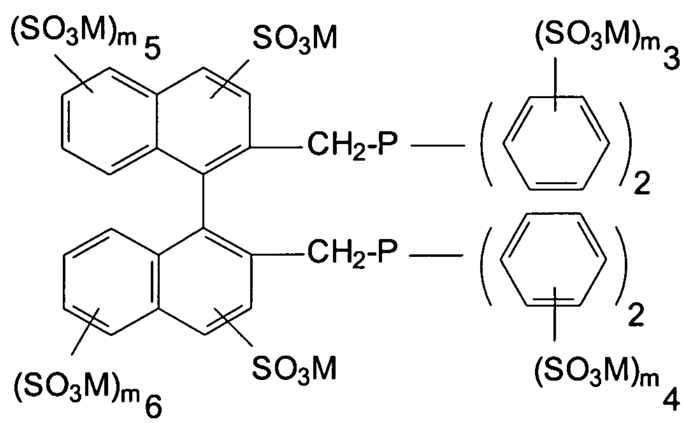

(12)

Diese Diphosphine der allgemeinen Formeln (11) und (12) sind aus W098/30526 bekannt.

In (11) steht ein jedes $m_1$ und $m_2$ unabhängig voneinander für 0 oder 1, wobei die Verbindung der Formel (11) bis sechs $-SO_3M$-Gruppe enthält.

In (12) steht ein jedes $m_3$, $m_4$, $m_5$ und $m_6$ unabhängig voneinander für 0 oder 1, wobei die Verbindung der Formel (12) vier bis acht $-SO_3M$-Gruppe enthält.

Aufgrund der Herstellung durch Sulfonierung der entsprechenden Diphosphine der Formeln (11a) und (12a), die keine $-SO_3M$-Gruppe enthalten,

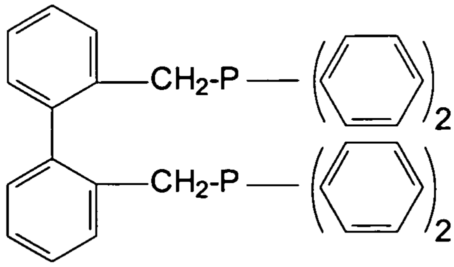

(11a)

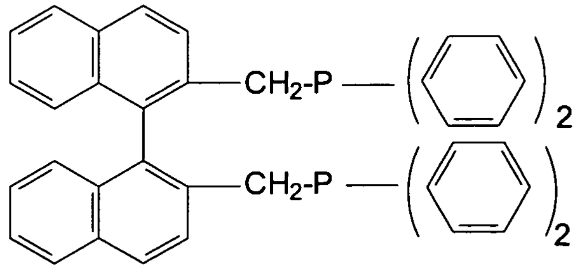

(12a)

erhält man üblicherweise Gemische von Verbindungen (11) und (12) mit unterschiedlicher Anzahl von $-SO_3M$-Gruppen. So enthält eine Verbindung der Formeln (11) oder (12), die beispielsweise drei $-SO_3M$-Gruppen enthält, auch Verbindungen mit lediglich zwei $-SO_3M$-Gruppen aber auch Verbindungen mit vier oder fünf $-SO_3M$-Gruppen Eine Verbindung der Formeln (11) oder (12) mit beispielsweise fünf $-SO_3M$-Gruppen enthält üblicherweise auch Verbindungen mit lediglich drei oder vier $-SO_3M$-Gruppe aber auch Verbindungen mit sechs oder sieben $-SO_3M$-Gruppen

Verbindungen der Formel (11) besitzen maximal sechs $-SO_3M$-Gruppen während Verbindungen der Formel (12) maximal acht $-SO_3M$-Gruppen aufweisen.

Aus diesem Grund gelangen in der Regel Mischungen von Verbindungen der Verbindungen (11) und (12) mit unterschiedlicher Anzahl von $-SO_3M$-Gruppen zum Einsatz.

In den Formeln (11) und (12) steht M für Ammonium, ein einwertiges Metall oder das Äquivalent eines mehrwertigen Metalls, insbesondere für Natrium, Kalium, Calcium oder Barium.

Als sulfonierte Arylphosphine eignen sich besonders sulfonierte Diphosphine, die sich von dem Xanthengerüst der allgemeinen Formel (13)

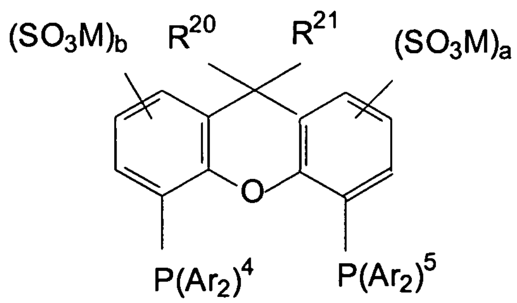

(13)

ableiten lassen, in der $R^{20}$ und $R^{21}$ gleich oder verschieden sind und einen linearen oder verzweigten Alkylrest mit 1-6 Kohlenstoffatomen oder einen Aralkylrest mit 7-14 Kohlenstoffatomen bedeuten, a und b gleich oder verschieden sind und 1, 2 oder 3 bedeuten, M für Ammonium, ein einwertiges Metall oder das Äquivalent eines mehrwertigen Metalls steht, insbesondere für Natrium, Kalium, Calcium oder Barium und $Ar^4$ und $Ar^5$ gleiche oder verschiedene Arylgruppen mit 6 bis 14 Kohlenstoffatomen bedeuten.

Vorzugsweise verwendet man solche D iphosphine der allgemeinen F ormel (13), in denen $Ar^4$ und $Ar^5$ Phenyl bedeuten, a und b gleich 1 sind und die $SO_3M$-Grupp sich in 2,8-Position, d.h. sich in meta-Stellung zum Phosphoratom befindet.

Die Phosphine auf Basis des Xanthengerüstes sind aus Organometallics 2000, Vol. 1 9, S eiten 872-883 b ekannt, a us d enen d urch S ulfonierung d ie sulfonierten Arylphosphine der allgemeinen Formel 13 erhalten werden.

Die Rhodium-Konzentration in der ionischen Flüssigkeit beträgt 10 bis 1000 Gew.-ppm, vorzugsweise 50 bis 500 Gew.-ppm, jeweils bezogen auf die ionische Flüssigkeit. Obgleich es möglich ist, als Katalysator die stöchiometrisch zusammengesetzte Rhodium-Phosphor-Komplexverbindung einzusetzen, arbeitet man üblicherweise in Gegenwart von überschüssigem Phosphorliganden, d.h. Ligand, der mit Rhodium keine komplexe Bindung eingegangen ist. Man bezeichnet auch die Rhodium-Phosphor-Komplexverbindung zusammen mit dem überschüssigen Liganden als Katalysatorsystem. Je mol Rhodium wendet man bevorzugt 2 bis 1000 mol Phosphor in Form der organischen Phosphor(III)-Verbindungen an. Besonders bewährt haben sich molare Verhältnisse von Rhodium zu Phosphor im Bereich von 1:5 bis 1:100. Der Rhodium-Phosphor-Komplexkatalysator braucht nicht einheitlich zusammengesetzt sein, sondern kann z.B. aus einem Gemisch von Rhodium-Komplexverbindungen bestehen, die sich durch die Art der Phosphorverbindungen unterscheiden.

Ebenso kann der in der ionischen Flüssigkeit enthaltene freie Phosphorligand aus einem Gemisch unterschiedlicher organischer Phosphor(III)-Verbindungen zusammengesetzt sein.

Die zweite Komponente des Katalysatorsystems, das Rhodium, kann entweder als Metall in feinverteilter Form, vorzugsweise auf einem Träger wie Aktivkohle, Calciumcarbonat, Tonerde oder ähnlichen Substraten oder als Rhodiumverbindung eingesetzt werden. Beispiele für anorganische oder organische Rhodiumverbindungen, in denen das Rhodium in seinen verschiedenen Oxidationsstufen vorliegen kann, sind die Rhodiumoxide $Rh_2O$, $Rh_2O_3$, $RhO_2$, $RhO_3$, die Salze der anorganischen Wasserstoffsäuren wie Halogenide, Sulfide, Selenide und Telluride, die Salze anorganischer Sauerstoffsäuren wie Rhodiumnitrat, Rhodiumsulfat, Rhodiumperchlorat, sowie die Salze aliphatischer Mono- oder Polycarbonsäuren wie Rhodiumacetat, Rhodiumpropionat, Rhodiumoxalat, Rhodiummalonat und Rhodium-2-ethylhexanoat. Weiterhin haben sich Carbonylverbindungen des Rhodiums wie Tricarbonylrhodium, $Rh(CO)_3$, Tetracarbonylrhodium, $[Rh(CO)_4]_2$, Tetrarhodiumdodekacarbonyl, $Rh_4(CO)_{12}$ und Rhodiumacetylacetonato dicarbonyl $[Rh(acac)(CO)_2]$ sehr bewährt. Halogencarbonylverbindungen wie Dicarbonylrhodiumbromid, $[Rh(CO)_2]Br$ und Dicarbonylrhodiumjodid, $[Rh(CO)_2]I$, können zwar auch eingesetzt werden, finden wegen des korrosiven Verhaltens der Halogenionen jedoch nur begrenzte Anwendung. Schließlich sind auch komplexe Verbindungen des Rhodiums, insbesondere Rhodium(III)-Verbindungen, geeignete Ausgangsmaterialien zur Herstellung der katalytisch aktiven Metallkomponente im Katalysatorsystem. Diese Verbindungen enthalten ein-, zwei- oder dreibindige Liganden wie β-Diketone, z.B. Acetylaceton, ferner Alkylamine, Alkyl- oder Aryldiamine, stickstoffhaltige Heterocyclen wie Pyridin oder aliphatische oder cycloaliphatische und diethylenisch ungesättigte Kohlenwasserstoffe wie Cyclopentadien und 1,5-Cyclooctadien. Für die Bildung des Katalysatorsystems besonders geeignete Rhodiumverbindungen sind die Rhodiumoxide, die Rhodiumcarbonyle, Rhodiumacetat, Rhodium-2-ethylhexanoat und Rhodium(III)-acetylacetonat.

Das Katalysatorsystem kann im Anfangsstadium der Umsetzung in situ, d.h. in der Reaktionsphase, unter Reaktionsbedingungen und in Gegenwart des Olefins aus den Komponenten Rhodium oder Rhodiumverbindung, organische Phosphorverbindung und Synthesegas hergestellt werden. Es ist aber auch möglich, das Katalysatorsystem getrennt von der Hydroformylierungsstufe in einem eigenen Reaktionsschritt zu präformieren und darauf dem Reaktionsgemisch zuzusetzen. Zur Präformierung suspendiert oder löst man metallisches Rhodium oder eine Rhodiumverbindung und organische Phosphorverbindung in der ionischen Flüssigkeit und behandelt das Gemisch mit Synthesegas. Typische Reaktionsbedingungen sind Temperaturen von 90 bis 150°C, insbesondere 100 bis 120°C und Drücke von 0,2 bis 10, vorzugsweise 0,5 bis 5 MPa. Die Reaktionszeit beträgt in Abhängigkeit von den gewählten Reaktionsbedingungen bis zu 5 h.

Die Umsetzung der Olefine bzw. der olefinisch ungesättigten Verbindungen mit Wasserstoff und Kohlenmonoxid zu Carbonylverbindungen erfolgt bei Temperaturen von 20 bis 150°C, bevorzugt 80 bis 140°C und insbesondere 100 bis 125°C und Drücken von 0,1 bis 20 MPa, vorzugsweise 0,5 bis 12 MPa und insbesondere 1 bis 7 MPa. Die im Einzelfall anzuwendenden Reaktionsbedingungen hängen auch von der Art der umzusetzenden olefinischen Verbindung ab. So lassen sich reaktionsfähige Einsatzstoffe bereits bei relativ niedrigen Temperaturen und Drücken und in Gegenwart geringer Katalysatormengen umsetzen, während reaktionsträgere Verbindungen entsprechend energischere Reaktionsbedingungen erfordern.

Die Zusammensetzung des Synthesegases, d.h. die Anteile von Kohlenmonoxid und Wasserstoff im Gasgemisch, kann in weiten Grenzen variiert werden. Im allgemeinen setzt man Gemische ein, in denen das Volumenverhältnis von Kohlenmonoxid zu Wasserstoff 5 : 1 bis 1 : 5 beträgt. Üblicherweise ist dieses Verhältnis 1 : 1 oder weicht von diesem Wert nur wenig ab.

Die olefinische Verbindung kann als solche oder in Lösung der Hydroformylierung zugeführt werden. Geeignete Lösungsmittel sind Ketone wie Aceton, Methylethylketon, Acetophenon, Dialkylether, wie Di-n-butylether, niedere aliphatische Nitrile wie Acetonitil, Propionitril oder Benzonitril, Dimethylformamid, lineare oder verzweigte gesättigte aliphatische Monohydroxyverbindungen wie Methanol, Ethanol, Propanol und Isopropanol, aromatische Kohlenwasserstoffe wie Benzol oder Toluol und gesättigte cycloaliphatische Kohlenwasserstoffe wie Cyclopentan oder Cylcohexan oder gesättigte aliphatische Kohlenwasserstoffe.

Das erfindungsgemäße Verfahren kann sowohl absatzweise als auch kontinuierlich durchgeführt werden. Nach Beendigung der Umsetzung erhält man zwei Phasen, das spezifisch leichtere Reaktionsprodukt als obere und die spezifisch schwerere Katalysatorlösung als untere Phase. Beide Stoffgemische lassen sich in einfacher Weise, z. B. durch Dekantieren, voneinander trennen.

Zur Erleichterung und Vervollständigung der Phasentrennung kann es sich als zweckmäßig erweisen, ein

unpolares organisches Lösungsmittel zuzusetzen. Als organische unpolare Lösungsmittel sind aliphatische, cycloaliphatische oder aromatische Kohlenwasserstoffe, insbesondere Pentan, Hexan, Cyclohexan, Heptan, Octan, i-Octan, Decan, Dodecan, Toluol, Xylol, Mesitylen oder Ethylbenzol geeignet. Nach Phasentrennung kann das Katalysatorsystem partiell oder vollständig in den Hydroformylierungsprozeß zurückgeführt werden. Die abgetrennte organische Phase wird in Lösungsmittel und Rohaldehyd destillativ aufgetrennt, wobei das Löungsmittel in den Phasentrennprozeß zurückgeführt werden kann und der Rohaldehyd weiteren Reinigungsprozessen oder einer nachfolgenden Umsetzung zugeleitet wird.

Die Anwendung des neuen Verfahrens ist nicht auf bestimmte Olefine als Ausgangsstoffe beschränkt. Dementsprechend können aliphatische, cycloaliphatische Verbindungen oder Verbindungen mit einem Alkylarylrest, die eine oder mehrere olefinische Doppelbindungen besitzen und gegebenenfalls auch noch funktionelle Gruppen enthalten, umgesetzt werden. Beispiele für aliphatische Verbindungen sind lineare oder verzweigte Olefine mit end- oder innenständigen Doppelbindungen wie Ethylen, Propylen, Buten-1, Buten-2, Isobuten, Penten-1, 2-Methylbuten-1, Hexen-1, Hepten-1, Octen-1, Octen-2, Octen-3, 2,4,4-Trimethylpenten-1, Nonen-1, 2-Propylhexen-1, Decen-1, Decen-3, Undecen-3, 4,4-Methylnonen-1, 6-Propyldecen-1. Auch konjugierte Polyolefine, wie z.B. Butadien-1.3 lassen sich mit Erfolg umsetzen. Als cycloaliphatische Einsatzstoffe kommen z.B. Dicyclopentadien, Vinylcyclohexen, Cyclooctadien und cyclische Terpene wie Limonen, und Pinen in Betracht. Beispiele für Olefine mit einem Alkylarylrest sind Styrol, $\alpha$-Methylstyrol, 1,1-Diphenylethylen, Divinylbenzol und m-Hexylstyrol.

Beispiele für olefinische Verbindungen mit funktionellen Gruppen sind Alkohole, Aldehyde, Carbonsäuren, Ester, Nitrile und Halogenverbindungen. Zu ihnen gehören Vinylverbindungen, insbesondere Ether und Ester wie Vinylmethylether, Vinylethylether, $\beta$-Vinylnaphthalin, o-Vinyl-p-xylol, Vinylacetat; Allylverbindungen, unter ihnen insbesondere die Alkohole und Ester wie Allylalkohol, Allyethylether und Allylacetat; Aldehyde wie Acrolein, Methacrolein, Crotonaldehyd; Ester der Acrylsäure, der Methacrylsäure, der Fumarsäure und der Maleinsäure; Acrylnitril. Diese Aufzählung geeigneter Ausgangsstoffe ist nicht erschöpfend, sondern lediglich beispielhaft.

Der erfindungsgemäße Prozeß eignet sich insbesondere zur Hydroformylierung wasserempfindlicher Olefine und Olefinderivate, wie die Ester des Vinylalkohols, z.B. Vinylacetat, Vinylpropionat, des Allylalkohols, wie Allylacetat, Allypropionat, Allylbutyrat, die Ester der Acrylsäure und die Acetale des Acroleins. Mit besonderem Erfolg lassen sich nach dem neuen Verfahren Olefine und Olefinderivate mit 2 bis 20 Kohlenstoffatomen hydroformylieren.

Die folgenden Beispiele sollen die Erfindung erläutern, diese aber nicht einschränken.

<u>Beispiele</u>

**1. Hydroformylierung von 1-Octen in BEIM-Tos**

Eine Katalysatorlösung enthaltend 5,3 mg $Rh(acac)(CO)_2$ und 40,0 mg 4,6-Bis(diphenylphosphino)-9,9'-dimethyl-2,8-dinatriumdisulfonatoxanthen in 5 mL 1-Butyl-3-ethylimidazolium-tosylat (BEIM-Tos) und 0,87 g Dibutylether als internem Standard wurde 0,5 h bei 120°C mit Synthesegas ($CO/H_2$ = 1:1) bei einem Gesamtdruck von 1,1 MPa im Autoklaven präformiert. Nach Präformierung wurden 2,24 g 1-Octen zugegeben und 2 h bei 1,1 MPa hydroformyliert. Der Reaktorinhalt wurde auf Raumtemperatur gekühlt, ausgebaut und die Phasen getrennt. Die organische Phase wurde gaschromatographisch analysiert und der Rhodiumgehalt per ICP-Messung (ICP = Inductive Coupled Plasma) bestimmt.

Umsatz: 56 %, n/i = 33:1, TOF = 270 $h^{-1}$ (turn-over-frequency), Summe an Hydrierungs- und Isomerisierungsprodukten: 8,4 %, [Rh] = 0,060 ppm (Rhodium-Konzentration in der abgetrennten organischen Phase).

**2. Hydroformylierung von 1-Octen in BMIM-$OcSO_4$**

Eine Katalysatorlösung enthaltend 5,1 mg $Rh(acac)(CO)_2$ und 38,5 mg 4,6-Bis(diphenylphosphino)-9,9'-dimethyl-2,8-dinatriumdisulfonatoxanthen in 5 mL 1-Butyl-3-methylimidazolium-octylsulfat (BMIM-$OcSO_4$ und 0,84 g Dibutylether als internem Standard wurde 0,5 h bei 120°C mit Synthesegas ($CO/H_2$ = 1:1) bei einem Gesamtdruck von 1,1 MPa im Autoklaven präformiert. Nach Präformierung wurden 2,28 g 1-Octen zugegeben und 2 h bei 1,1 MPa hydroformyliert. Der Reaktorinhalt wurde auf Raumtemperatur gekühlt, ausgebaut und zur Phasentrennung wurden 6 mL Cyclohexan zugegeben. Die organische Phase wurde gaschromatographisch a nalysiert und der Rhodiumgehalt per ICP-Messung bestimmt.

Umsatz: 63 %, n/i = 49:1, TOF = 323 $h^{-1}$, Summe an Hydrierungs- und Isomerisierungsprodukten: 10,0 %, [Rh] = 0,368 ppm.

Vergleichsbeispiel

**Hydroformylierung von 1-Octen in BMIM-$PF_6$**

Eine Katalysatorlösung enthaltend 5,1 mg Rh(acac)$(CO)_2$ und 38,1 mg 4,6-Bis(diphenylphosphino)-9,9'-dimethyl-2,8-dinatriumdisulfonatoxanthen in 5 mL 1-Butyl-3-methylimidazolium-hexafluorophosphat (BMIM-$PF_6$) und 0,86 g Dibutylether als internem Standard wurde 0,5 h bei 120°C mit Synthesegas ($CO/H_2$ = 1:1) bei einem Gesamtdruck von 1,1 MPa im Autoklaven präformiert. Nach Präformierung wurden 2,37 g 1-Octen zugegeben und 2 h bei 1,1 MPa hydroformyliert. Der Reaktorinhalt wurde auf Raumtemperatur gekühlt, ausgebaut und die Phasen getrennt. Die organische Phase wurde gaschromatographisch analysiert und der Rhodiumgehalt per ICP-Messung bestimmt.

Umsatz: 17 %, n/i = 33:1, TOF = 85 $h^{-1}$, Summe an Hydrierungs- und Isomerisierungsprodukten: 3,0 %, [Rh] = 0,027 ppm.

Wie die Ergebnisse der erfindungsgemäßen Beispiele zeigen, lassen sich deutlich höhere Umsatz-, Selektivitäts- und TOF-Werte in der Hydroformylierungsreaktion beobachten, wenn man anstelle der bekannten ionischen Flüssigkeiten auf Basis von komplexen Halogen haltigen Anionen solche ionische Flüssigkeiten verwendet, die aus organischen Sulfonaten oder Sulfaten als Anionen aufgebaut sind. Auch der Rhodiumaustrag aus der ionischen Flüssigkeit in die organische Phase ist gering.

## Patentansprüche

1. Verfahren zur Herstellung von Aldehyden durch Umsetzung von Monoolefinen, konjugierten und nichtkonjugierten Polyolefinen, Cycloolefinen oder Derivaten dieser Verbindungsklassen mit Kohlenmonoxid und Wasserstoff bei Temperaturen von 20 bis 150°C und Drücken von 0,1 bis 20 MPa in Gegenwart einer nicht wäßrigen ionischen Flüssigkeit der allgemeinen Formel $(Q^{\oplus})_a A^{a-}$ und mindestens einer Rhodiumverbindung und mindestens eines sulfonierten Arylphosphins, **dadurch gekennzeichnet, daß** $Q^{\oplus}$ ein einfach geladenes, gegebenenfalls durch organische Reste substituiertes Ammonium-Kation oder das Äquivalent eines mehrfach geladenen, gegebenenfalls durch organische Reste substituierten Ammonium-Kations ist und $A^{a-}$ für ein organisches Sulfonat oder organisches Sulfat der allgemeinen Formeln

$$R^1 \{ SO_3^- )_a \text{ oder } R^2 \{ OSO_3^- )_a \qquad (1)$$

steht, wobei $R^1$ und $R^2$ einen geradkettigen oder verzweigten Alkylrest mit 1-20 Kohlenstoffatomen, einen Alkylarylrest mit 7-20 Kohlenstoffatomen, einen Arylrest mit 6-14 Kohlenstoffatomen, einen Cycloalkylrest mit 3-7 Kohlenstoffatomen oder einen Aralkylrest mit 7-20 Kohlenstoffatomen bedeutet; oder $R^1$ einen Rest der allgemeinen Formel

$$R^3\text{- O}\{(CH_2\}_n O\}_m \qquad (1a)$$

bedeutet, in der $R^3$ einen geradkettigen oder verzweigten Alkylrest mit 1-20 Kohlenstoffatomen, einen Alkylarylrest mit 7-20 Kohlenstoffatomen, einen Arylrest mit 6-14 Kohlenstoffatomen, einen Cycloalkylrest mit 3-7 Kohlenstoffatomen oder einen Aralkylrest mit 7-20 Kohlenstoffatomen darstellt, n eine ganze Zahl von 2-12 bedeutet und m ganzzahlige Werte von 1-100 annimmt; und a eine ganze Zahl ist und die Anzahl der an dem organischen Rest gebundenen Sulfon- oder Sulfatreste bedeutet und mindestens gleich 1 ist.

2. Verfahren nach Anspruch 1, **dadurch gekennzeichnet, dass** $R^1$ oder $R^2$ für einen geradkettigen oder verzweigten Alkylrest mit 1-12 Kohlenstoffatomen, einen Alkylarylrest mit 7-12 Kohlenstoffatomen, einen Arylrest mit 6-10 Kohlenstoffatomen, einen Cycloalkylrest mit 5-7 Kohlenstoffatomen oder einen Aralkylrest mit 7-12 Kohlenstoffatomen steht, $R^3$ einen geradkettigen oder verzweigten Alkylrest mit 1-12 Kohlenstoffatomen, einen Alkylarylrest mit 7-12 Kohlenstoffatomen, einen Arylrest mit 6-10 Kohlenstoffatomen, einen Cycloalkylrest mit 5-7 Kohlenstoffatomen oder einen Aralkylrest mit 7-12 Kohlenstoffatomen bedeutet, n gleich 2, 3 oder 4 ist, m eine ganze Zahl zwischen 1-50 ist; und a gleich 1 oder 2 ist.

3. Verfahren nach Anspruch 1 oder 2, **dadurch gekennzeichnet, dass** das organische Sulfonat für Tosylat und das organische Sulfat für Octylsulfat steht.

**4.** Verfahren nach einem oder mehreren der Ansprüche 1 bis 3, **dadurch gekennzeichnet, dass** $Q^{\oplus}$ für Ammoniumionen der allgemeinen Formeln (2) und (3) stehen

$$^{\oplus}NR^4R^5R^6R^7 \quad (2)$$

und

$$R^4R^5N^{\oplus} = CR^6R^7 \quad (3)$$

wobei $R^4$, $R^5$, $R^6$ und $R^7$ gleich oder verschieden sind und Wasserstoff, insbesondere mit der Maßgabe, daß mindestens ein $R^4$, $R^5$, $R^6$, $R^7$ nicht Wasserstoff ist, oder einen linearen oder verzweigten, aliphatischen Kohlenwasserstoffrest mit 1 bis 20 Kohlenstoffatomen, einen cycloaliphatischen oder aromatischen Kohlenwasserstoffrest mit 6 bis 20 Kohlenstoffatomen oder einen Alkoxyrest mit 1 bis 10 Kohlenstoffatomen bedeuten.

**5.** Verfahren nach einem oder mehreren der Ansprüche 1 bis 3, **dadurch gekennzeichnet, dass** $Q^{\oplus}$ für gesättigte oder ungesättigte cyclische Verbindungen, oder für aromatische Verbindungen mit jeweils einem dreibindigen Stickstoffatom im 4- bis 10-gliedrigen heterocyclischen Ring der allgemeinen Formeln (4) und (5) stehen

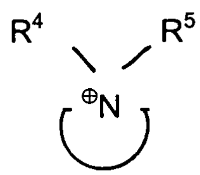

(4)

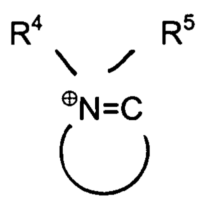

(5)

wobei $R^4$ und $R^5$ gleich oder verschieden sind und Wasserstoff, insbesondere mit der Maßgabe, dass mindestens ein $R^4$, $R^5$, $R^6$, $R^7$ nicht Wasserstoff ist, oder einen linearen oder verzweigten, aliphatischen Kohlenwasserstoffrest mit 1 bis 20 Kohlenstoffatomen, einen cycloalipahtischen oder aromatischen Kohlenstoffrest mit 6 bis 20 Kohlenstoffatomen oder einen Alkoxyrest mit 1 bis 10 Kohlenstoffatomen bedeuten.

**6.** Verfahren nach einem oder mehreren der Ansprüche 1 bis 3, **dadurch gekennzeichnet, dass** $Q^{\oplus}$ für quatäre Ammoniumionen der allgemeinen Formeln (6) oder (7) stehen

$$R^4R^5R^6N^{\oplus}\text{-G-}N^{\oplus}R^7R^8\ R^9 \quad (6)$$

$$R^4R^5N^{\oplus} = CR^6\text{-G-}R^6C = N^{\oplus}R^4R^5 \quad (7)$$

in denen $R^4$, $R^5$, $R^6$, $R^7$, $R^8$ und $R^9$ gleich oder verschieden sind und Wasserstoff, einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 30 Kohlenstoffatomen, einen Alkylaryl- oder Aralkylrest mit 7 bis 40 Kohlenstoffatomen oder einen Alkoxyrest mit 1 bis 10 Kohlenstoffatomen bedeuten; G für einen Alkylenrest (-$CHR^{10}$-)$_d$ steht, wobei $R^{10}$ Wasserstoff oder ein Kohlenwasserstoffrest mit 1 bis 5 Kohlenstoffatomen und d eine ganze Zahl von 1 bis 8, vorzugsweise 2 bis 6 ist, für einen Arylenrest mit 6 bis 30 Kohlenstoffatomen oder für einen Alkylenarylrest mit 7 bis 40 Kohlenstoffatomen steht.

**7.** Verfahren nach einem oder mehreren der Ansprüche 1 bis 3, **dadurch gekennzeichnet, dass** $Q^{\oplus}$ für Imidazoliumionen der allgemeinen Formel (9) steht

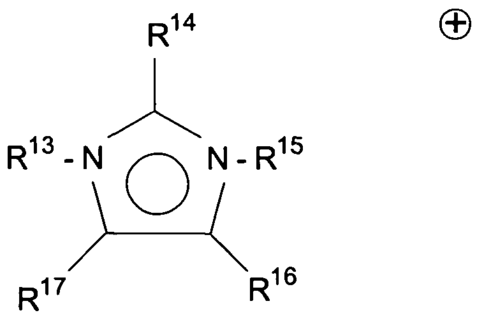

(9)

in der $R^{13}$, $R^{14}$, $R^{15}$, $R^{16}$ und $R^{17}$ gleich oder verschieden sind und für Wasserstoff, einen $C_1$- bis $C_{30}$-Alkylrest, einen $C_6$- bis $C_{40}$-Arylrest, einen $C_7$- bis $C_{40}$-Alkylarylrest oder einen $SiR_3{}^{18}$-Rest steht, in dem $R^{18}$ einen $C_1$- bis $C_{30}$-Alkylrest oder einen $C_6$- bis $C_{40}$-Arylrest bedeutet.

**8.** Verfahren nach Anspruch 7, **dadurch gekennzeichnet, dass** $Q^{\oplus}$ ausgewählt wird aus der Gruppe 1-Ethyl-3-methyl-2,4,5-H-imidazolium, 1-Propyl-3-methyl-2,4,5-H-imidazolium, 1-Butyl-3-methyl-2,4,5-Himidazolium, 1-Butyl-3-ethyl-2,4,5-H-imidazolium, 1,3,4,5-Tetramethyl-2-H-imidazolium, 2,4,5-Trimethyl-1,3-H-imidazolium, 1,2,3,4,5-Pentamethylimidazolium, 1,2,3,5-Tetramethyl-4-H-imidazolium, 1,2,3,4-Tetramethyl-5-H-imidazolium, 1,3,4,5-Tetraphenyl-2-H-imidazolium, 1,3-Dimethyl-4,5-diphenyl-2-H-imidazolium, 1-Ethyl-3-isopropyl-2,4,5-Himidazolium, 1-Butyl-3-octanyl-2,4,5-H-imidazolium, 1-Propyl-3-octanyl-2,4,5-H-imidazolium, 1-Ethyl-3-octanyl-2,4,5-H-imidazolium, 1-Methyl-3-octanyl-2,4,5-H-imidazolium, 1,3-Diisoproypl-4,5-dimethyl-2-Himidazolium, 1,4,5-Trimethyl-3-trimethylsilyl-2-H-imidazolium, 2-Ethyl-4-methyl-1,3,5-H-imidazolium, 1,3-Adamantyl-4,5-dimethyl-1-Himidazolium, 1,2,4,5-Tetramethyl-3-H-imidazolium, 1-Methyl-2,3,4,5-Himidazolium, 1,3-Dimethyl-2,4,5-H-imidazolium, 2-Methyl-4,5-ethyl-1,3-H-imidazolium, 2,4,5-Trimethyl-1,3-H-imidazolium, 1-Ethyl-2,3,4,5-Himidazolium, 1,3-Diethyl-4,5-dimethyl-2-H-imidazolium, 1,3-Diphenyl-4,5-dimethyl-2-H-imidazolium, 1,3-Diphenyl-2,4,5-H-imidazolium, 1,3-Dimethoxy-4,5-dimethyl-2-H-imidazolium, 1-Trimethylsilyl-2,3,5-trimethyl-4-H-imidazolium.

**9.** Verfahren nach einem oder mehreren der Ansprüche 1 bis 8, **dadurch gekennzeichnet, dass** das sulfonierte Arylphosphin für ein sulfoniertes Triarylphosphin der allgemeinen Formel (10) steht

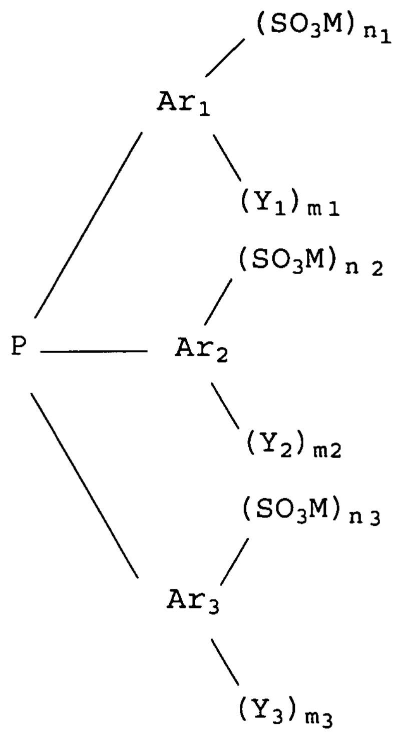

(10)

in der $Ar^1$, $Ar^2$ und $Ar^3$ gleiche oder verschiedene Arylgruppen mit 6 bis 14 Kohlenstoffatomen bedeuten, die Substituenten $Y_1$, $Y_2$ und $Y_3$ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel $NR^{18}R^{19}$ stehen, in der die Substituenten $R^{18}$ und $R^{19}$ gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der $m_1$, $m_2$ und $m_3$ gleich oder verschieden sind und ganze Zahlen von 0 bis 5 bedeuten, in der $n_1$, $n_2$ und $n_3$ gleich oder verschieden sind und für ganze Zahlen von 0 bis 3 stehen, wobei mindestens eine der Zahlen $n_1$, $n_2$ und $n_3$ gleich oder größer 1 ist.

**10.** Verfahren nach einem oder mehreren der Ansprüche 1 bis 8, **dadurch gekennzeichnet, dass** das sulfonierte Arylphosphin für ein sulfoniertes Diarylphosphin der allgemeinen Formel (11) steht

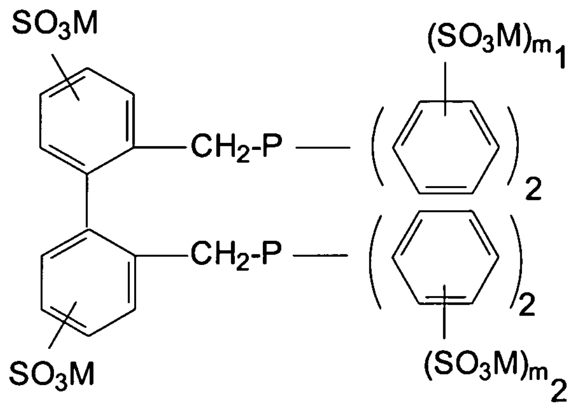

(11)

in der $m_1$ und $m_2$, unabhängig voneinander, für 0 oder 1 steht, wobei die Verbindung der Formel (11) bis sechs $-SO_3M$-Gruppe enthält und wobei M für Ammonium, ein einwertiges Metall oder das Äquivalent eines mehrwertigen Metalls, insbesondere für Natrium, Kalium, Calcium oder Barium steht.

**11.** Verfahren nach einem oder mehreren der Ansprüche 1 bis 8, **dadurch gekennzeichnet, dass** das sulfonierte Arylphosphin für ein sulfoniertes Diarylphosphin der allgemeinen Formel (12) steht

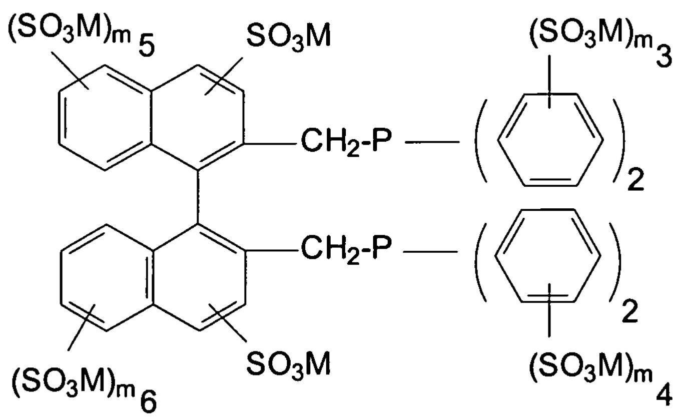

(12)

in der $m_3$, $m_4$, $m_5$ und $m_6$ unabhängig voneinander für 0 oder 1 steht, wobei die Verbindung der Formel (12) vier bis acht $-SO_3M$-Gruppe enthält, u nd wobei M für A mmonium, ein e inwertiges Metall o der d as Äquivalent eines mehrwertigen Metalls, insbesondere für Natrium, Kalium, Calcium oder Barium steht.

**12.** Verfahren nach einem oder mehreren der Ansprüche 1 bis 8, **dadurch gekennzeichnet, dass** das sulfonierte Arylphosphin für ein sulfoniertes Diarylphosphin der allgemeinen Formel (13) steht

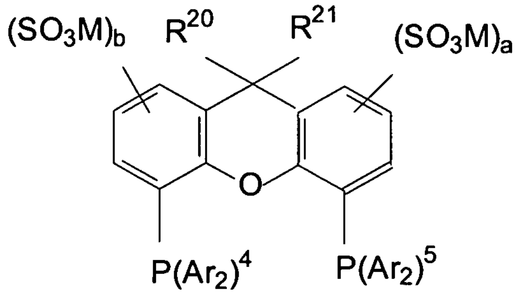

(13)

in der $R^{20}$ und $R^{21}$ gleich oder verschieden sind und einen linearen oder verzweigten Alkylrest mit 1-6 Kohlenstoffatomen oder einen Aralkylrest mit 7-14 Kohlenstoffatomen bedeuten, a und b gleich oder verschieden sind und 1, 2 oder 3 bedeuten, M für Ammonium, ein einwertiges Metall oder das Äquivalent eines mehrwertigen Metalls steht, insbesondere für Natrium, Kalium, Calcium oder Barium und $Ar^4$ und $Ar^5$ gleiche oder verschiedene Arylgruppen mit 6 bis 14 Kohlenstoffatomen bedeuten.

**13.** Verfahren nach einem oder mehreren der Ansprüche 1 bis 12, **dadurch gekennzeichnet, dass** je mol Rhodium 2 bis 1000, bevorzugt 5 bis 100 mol Phosphor(III) eingesetzt werden.

**14.** Verfahren nach einem oder mehreren der Ansprüche 1 bis 13, **dadurch gekennzeichnet, dass** die Rhodiumkonzentration 10 bis 1000 Gew.ppm, vorzugsweise 50 bis 500 Gew.-ppm, bezogen auf die ionische Flüssigkeit, beträgt.

**15.** Verfahren nach einem oder mehreren der Ansprüche 1 bis 14, **dadurch gekennzeichnet, dass** die Umsetzung bei 80 bis 140°C, vorzugsweise 100 bis 125°C erfolgt.

**16.** Verfahren nach einem oder mehreren der Ansprüche 1 bis 15, **dadurch gekennzeichnet, dass** die Umsetzung bei Drücken von 0,5 bis 12 MPa und insbesondere 1 bis 7 MPa erfolgt.

**17.** Verfahren nach einem oder mehreren der Ansprüche 1 bis 16, **dadurch gekennzeichnet, dass** Olefine oder Olefinderivate mit 2 bis 20 Kohlenstoffatomen eingesetzt werden.

**18.** Verfahren nach einem oder mehreren der Ansprüche 1 bis 17, **dadurch gekennzeichnet, dass** die organische aldehydhaltige Phase und die rhodiumhaltige ionische Flüssigkeit durch Phasentrennung voneinander getrennt werden und die abgetrennte rhodiumhaltige ionische Flüssigkeit vollständig oder teilweise in den Hydroformylierungsreaktor zurückgeführt wird.

**19.** Verfahren nach Anspruch 18, **dadurch gekennzeichnet, dass** man während der Phasentrennung ein unpolares organisches Lösungsmittel zusetzt.

Europäisches Patentamt

# EUROPÄISCHER RECHERCHENBERICHT

Nummer der Anmeldung
EP 03 02 0385

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| X | US 2002/115892 A1 (MACKEWITZ THOMAS) 22. August 2002 (2002-08-22) * Seite 7, Spalte 1, Zeile 5 - Zeile 13; Anspruch 2 * | 1-3,7,8, 12 | C07C45/50 C07C47/02 |
| X | WASSERSCHEID, PETER ET AL: "1-n-Butyl-3-methylimidazolium ([bmim]) octylsulfate - an even 'greener' ionic liquid" GREEN CHEMISTRY (2002), 4(4), 400-404 , XP008024341 * das ganze Dokument * | 1-3,7,8, 17-19 | |
| P,X | WO 03 022812 A (SOLVENT INNOVATION GMBH) 20. März 2003 (2003-03-20) * Ansprüche 1,8; Beispiel 2 * | 1-5,7 | |
| D,Y | EP 0 776 880 A (INST FRANCAIS DU PETROL) 4. Juni 1997 (1997-06-04) * das ganze Dokument * | 1-19 | |
| | | | RECHERCHIERTE SACHGEBIETE (Int.Cl.7) |
| Y | EP 1 182 197 A (SOLVENT INNOVATION GMBH) 27. Februar 2002 (2002-02-27) * Anspruch 1; Beispiele 10-13 * | 1-19 | C07C |
| D,Y | WASSERSCHEID, PETER ET AL: "Ionische Flüssigkeiten - neue "Lösungen" für die Übergangsmetallkatalyse" ANGEWANDTE CHEMIE, Bd. 112, 2000, Seiten 3927-3945, XP002261498 weinheim * Seite 3929 * | 1-19 | |
| Y | EP 1 241 156 A (INST FRANCAIS DU PETROL) 18. September 2002 (2002-09-18) * Seite 3, Zeile 28 - Seite 5; Anspruch 1 * | 1-19 | |
| | -/-- | | |

Der vorliegende Recherchenbericht wurde für alle Patentansprüche erstellt

| Recherchenort | Abschlußdatum der Recherche | Prüfer |
|---|---|---|
| DEN HAAG | 14. November 2003 | Bonnevalle, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 03 02 0385

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,Y | DE 26 27 354 A (RHONE POULENC IND)<br>23. Dezember 1976 (1976-12-23)<br>* Anspruch 1 *<br>--- | 1-19 | |
| D,Y | WO 98 30526 A ( HOECHST AG )<br>16. Juli 1998 (1998-07-16)<br>* Ansprüche 1,13,14 *<br>--- | 1-19 | |
| Y | MUL, WILHELMUS P. ET AL: "New, highly efficient work-up protocol for sulfonated diphosphines"<br>ADVANCED SYNTHESIS & CATALYSIS (2002), 344(3+4), 293-298 ,<br>XP002261499<br>* Seite 294 *<br>--- | 1-19 | |
| E | WO 03 074494 A (SOLVENT INNOVATION GMBH)<br>12. September 2003 (2003-09-12)<br>* Seite 18 - Seite 19; Ansprüche 1,8; Beispiel 1 *<br>----- | 1,7-9,<br>15-19 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| DEN HAAG | 14. November 2003 | Bonnevalle, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 03 02 0385

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-11-2003

| Im Recherchenbericht angeführtes Patentdokument | Datum der Veröffentlichung | Mitglied(er) der Patentfamilie | Datum der Veröffentlichung |
|---|---|---|---|
| US 2002115892 A1 | 22-08-2002 | DE 10206697 A1 | 29-08-2002 |
| | | JP 2002308817 A | 23-10-2002 |
| WO 03022812 A | 20-03-2003 | DE 10145747 A1 | 03-04-2003 |
| | | WO 03022812 A1 | 20-03-2003 |
| EP 0776880 A | 04-06-1997 | FR 2741875 A1 | 06-06-1997 |
| | | DE 69606689 D1 | 23-03-2000 |
| | | DE 69606689 T2 | 26-10-2000 |
| | | EP 0776880 A1 | 04-06-1997 |
| | | ES 2145409 T3 | 01-07-2000 |
| | | JP 9221443 A | 26-08-1997 |
| | | US 5874638 A | 23-02-1999 |
| EP 1182197 A | 27-02-2002 | EP 1182197 A1 | 27-02-2002 |
| EP 1241156 A | 18-09-2002 | FR 2819806 A1 | 26-07-2002 |
| | | EP 1241156 A1 | 18-09-2002 |
| | | JP 2002275117 A | 25-09-2002 |
| | | US 2002099243 A1 | 25-07-2002 |
| DE 2627354 A | 23-12-1976 | FR 2314910 A1 | 14-01-1977 |
| | | FR 2349562 A2 | 25-11-1977 |
| | | BE 843177 A1 | 20-12-1976 |
| | | BR 7603941 A | 05-04-1977 |
| | | CA 1074337 A1 | 25-03-1980 |
| | | CA 1152105 B | 16-08-1983 |
| | | DE 2627354 A1 | 23-12-1976 |
| | | ES 449003 A1 | 16-11-1977 |
| | | GB 1540242 A | 07-02-1979 |
| | | IT 1069268 B | 25-03-1985 |
| | | JP 57167937 A | 16-10-1982 |
| | | JP 1280528 C | 13-09-1985 |
| | | JP 52012110 A | 29-01-1977 |
| | | JP 59012091 B | 21-03-1984 |
| | | NL 7606634 A ,B, | 22-12-1976 |
| | | US RE31812 E | 22-01-1985 |
| | | US 4248802 A | 03-02-1981 |
| WO 9830526 A | 16-07-1998 | DE 19700804 C1 | 06-08-1998 |
| | | AT 216691 T | 15-05-2002 |
| | | CN 1247527 A | 15-03-2000 |
| | | DE 59707120 D1 | 29-05-2002 |
| | | DK 958270 T3 | 05-08-2002 |
| | | WO 9830526 A1 | 16-07-1998 |
| | | EP 0958270 A1 | 24-11-1999 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 03 02 0385

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-11-2003

| Im Recherchenbericht angeführtes Patentdokument | Datum der Veröffentlichung | Mitglied(er) der Patentfamilie | Datum der Veröffentlichung |
|---|---|---|---|
| WO 9830526 A | | ES 2175516 T3 | 16-11-2002 |
| | | JP 2002511055 T | 09-04-2002 |
| | | PT 958270 T | 30-09-2002 |
| | | TW 467890 B | 11-12-2001 |
| | | US 6274774 B1 | 14-08-2001 |
| | | ZA 9800212 A | 02-09-1998 |
| WO 03074494 A | 12-09-2003 | DE 10208822 A1 | 11-09-2003 |
| | | WO 03074494 A1 | 12-09-2003 |

EPO FORM P0461

Für nähere Einzelheiten zu diesem Anhang : siehe Amtsblatt des Europäischen Patentamts, Nr.12/82